# EUROPEAN PATENT APPLICATION

(11) **EP 1 477 922 A2**
(43) Date of publication of application: **17.11.2004**
(21) Application number: 04011300.3
(22) Date of filing: 12.05.2004
(51) Int. Cl.: G06F 19/00

(54) **Image management method, apparatus and program**

(30) Priority: 13.05.2003 JP 2003134737
(71) Applicant: CANON KABUSHIKI KAISHA, Ohta-ku, Tokyo (JP)
(72) Inventor: Matsuno, Hiroyuki, Tokyo (JP)
(74) Representative: Leson, Thomas Johannes Alois, Dipl.-Ing.

(57) **Abstract**

Disclosed are an image management method, apparatus and program through which shot images can be managed in ideal fashion without outputting unnecessary film even in a case where re-shooting is performed a number of times in regard to a shoot order. Management of shot images in a medical digital X-ray photography apparatus (4) includes acquiring a shot image of an imaged subject, instructing the image sensing device to re-shoot the image of the subject, acquiring the re-shot image of the subject that has been imaged by the image sensing apparatus, and storing the acquired shot image and the re-shot image on a hard disk (12) in correlated form. Management further includes adopting the shot image and the re-shot image that have been stored on the hard disk (12) as an invalid image and a valid image, respectively, and outputting the valid image from the hard disk (12) to an external device or the like.

## Description

### FIELD OF THE INVENTION

The present invention is directed to an image management technique for managing an X-ray digital image captured using a solid-state image sensing device.

### BACKGROUND OF THE INVENTION

A film screen system that is a combination of sensitized paper and X-ray photographic film is in wide use in X-ray photography for the purpose of medical diagnosis. The film image obtained by X-ray photography usually is observed by being hung from a view box. To facilitate observation of the area under diagnosis, the film image is set so as to obtain a contrast on the order of 1.0 to 1.5D (where D represents density), which is a density region in which observation is easy. If the shooting conditions in X-ray photography shift, however, there are instances where over- or under-exposure tends to occur, and this can have a deleterious effect upon diagnosis.

Further, the radiological shoot ordered by the physician is returned to the physician as X-ray film and the film is used in diagnosis. Film for which the shoot has failed is discarded as film of a rejected shoot. There is a need to reduce such wasting of film. In a case where an output is delivered in the form of film, it is difficult to establish correlation between the image that was shot originally and an image that has been re-shot, and orders for such radiological shoots cannot be managed appropriately.

An X-ray digital photography apparatus that employs an FPD (Flat Panel Detector) for effecting a conversion to an electric signal proportional to X-ray intensity has started being used in recent years. Using this photography apparatus makes it possible to solve the problem of exposure adjustment encountered in the prior art. Further, since the shot image can be checked immediately after shooting, it is possible to immediately confirm after shooting, without waiting for development of film, whether blurring of the subject due to unintentional movement of the patient has occurred. This makes it possible to reduce wasting of film. (By way of example, see the specification of Japanese Patent Application Laid-Open No. 10-262961.) As a result, whether or not a shot has failed can be determined immediately and re-shooting of a shoot order that has failed can be performed promptly. This greatly alleviates the burden of waiting time upon both the patient and the radiological technician. (By way of example, see the specification of Japanese Patent Application Laid-Open No. 11-195105.)

Even when the conventional X-ray digital photography apparatus is used, however, it is difficult to correlate the originally shot image and a re-shot image owing to the presence of rejected images. Consequently, shoot orders cannot be managed appropriately just as in the case of output in the form of film.

### SUMMARY OF THE INVENTION

The present invention has been proposed to solve the problems of the prior art and its object is to provide an image management method, apparatus and program through which shot images can be managed in ideal fashion without outputting unnecessary film even in a case where re-shooting is performed a number of times in regard to a shoot order.

According to the present invention, the foregoing object is attained by providing an image management method characterized by comprising a first acquisition step of acquiring a shot image of a subject that has been imaged by an image sensing device, an command step of commanding the image sensing device to re-shoot the image of the subject, a second acquisition step of acquiring the re-shot image of the subject imaged by the image sensing device, a storage step of storing the shot image, which has been acquired at the first acquisition step, and the re-shot image, which has been acquired by the second acquisition step, in correlated form in a storage device, a status setting step of adopting the shot image and the re-shot image, which have been stored in the storage device, as an invalid image and a valid image, respectively and an output step of outputting the valid image from the storage device.

Furthermore, according to the present invention, the foregoing object is attained by providing the method, characterized in that the storage step stores the shot and re-shot images in the storage device on a per-shoot-command basis at the command step using a hierarchical structure in which information relating to the shoot command is made a higher-order layer while the shot image and re-shot image are made a layer subordinate to information relating to the shoot command, the shot and re-shot images being in the same layer as each other.

Furthermore, according to the present invention, the foregoing object is attained by providing the method, characterized by further comprising a display step of displaying the shot image or re-shot image in the order of shoot commands at the command step.

Furthermore, according to the present invention, the foregoing object is attained by providing the method, characterized in that in a case where a plurality of images that include shot and re-shot images in a prescribed hierarchical layer situated subordinate to the same shoot command have been stored in the storage device, the display step displays the plurality of images in order before images relating to the next shoot command or the shoot sequence are displayed.

Furthermore, according to the present invention, the foregoing object is attained by providing the method, characterized in that the status setting step causes an invalidity flag indicative of an invalid image or a validity flag indicative of a valid image to be retained as information attached to the shot and re-shot images.

Furthermore, according to the present invention, the foregoing object is attained by providing the method, characterized by further comprising a mark add-on step of adding an invalidity mark onto the shot image if re-shooting of the subject has been command by the command step, characterized in that the storage step stores the shot image having the added-on invalidity mark in correlation with the re-shot image.

Furthermore, according to the present invention, the foregoing object is attained by providing the method, characterized in that the status setting step adopts the shot image having the added-on invalidity mark as an invalid image, adopts the re-shot image, which has been acquired by the second acquisition step, as a valid image, and erases neither the invalid image nor the valid image.

Furthermore, according to the present invention, the foregoing object is attained by providing the method, characterized by further comprising an information add-on step of adding on information, which indicates that the valid image is a re-shot image, to the valid image.

Furthermore, according to the present invention, the foregoing object is attained by providing the method, characterized by further comprising an auxiliary-information display step which, when the valid image is displayed by the display step, is a step of displaying auxiliary information, which indicates the absence or presence of an invalid image corresponding to the valid image, in the valid image or together with the display of the valid image.

Furthermore, according to the present invention, the foregoing object is attained by providing an image management apparatus characterized by comprising shooting means for shooting a subject and acquiring a shot image of the subject, command means for commanding the image sensing device to re-shoot the image of the subject, re-shooting means for re-shooting the subject and acquiring a re-shot image of the subject based upon a command from the command means, storage means for storing the shot image and the re-shot image in correlated form, status setting means for adopting the shot image and the re-shot image, which have been stored in the storage means, as an invalid image and a valid image, respectively and output means for outputting the valid image from the storage device.

Furthermore, according to the present invention, the foregoing object is attained by providing a program for causing a computer to execute the following procedures: a first acquisition procedure for acquiring a shot image of a subject that has been imaged by an image sensing device, an command procedure for commanding the image sensing device to re-shoot the image of the subject, a second acquisition step of acquiring the re-shot image of the subject imaged by the image sensing device, a storage procedure for storing the shot image, which has been acquired at the first acquisition step and the re-shot image, which has been acquired by the second acquisition step, in correlated form in a storage device, a status setting procedure for adopting the shot image and the re-shot image, which have been stored in the storage device, as an invalid image and a valid image, respectively and an output procedure for outputting the valid image from the storage device.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

The accompanying drawings, which are incorporated in and constitute a part of the specification, illustrate embodiments of the invention and, together with the description, serve to explain the principles of the invention.
Fig. 1 is a block diagram illustrating the configuration of an X-ray image photography system according to an embodiment of the present invention;
Fig. 2 is a diagram illustrating an example of display of a list of shoot orders displayed on a display unit according to this embodiment;
Fig. 3 is a diagram illustrating an example of display of a shoot preparation screen displayed on the display unit according to this embodiment;
Fig. 4 is a diagram illustrating an example of a post-shoot display screen displayed on the display unit according to this embodiment;
Fig. 5 is a diagram illustrating an example of a display screen showing the state of a rejected image after re-shooting;
Fig. 6 is a diagram useful in describing an example of image management before re-shooting by an ordinary digital photography apparatus according to the prior art;
Fig. 7 is a diagram useful in describing an example of image management in a case where re-shooting has been performed with regard to "CHEST; FRONT" of a first shoot order;
Fig. 8 is a diagram useful in describing an example of image management in case of a fourth re-shoot with regard to "CHEST; FRONT" of a first re-shoot order;
Fig. 9 is a diagram useful in describing an example of image management after re-shooting is performed according to this embodiment;
Fig. 10 is a flowchart for describing the procedure of re-shoot processing in response to a re-shoot command; and
Fig. 11 is a flowchart for describing the procedure of image management in response to operation of a reject button 46.

### DETAILED DESCRIPTION OF THE PREFERRED EMBODIMENT

An embodiment of the present invention will now be described with reference to the drawings.

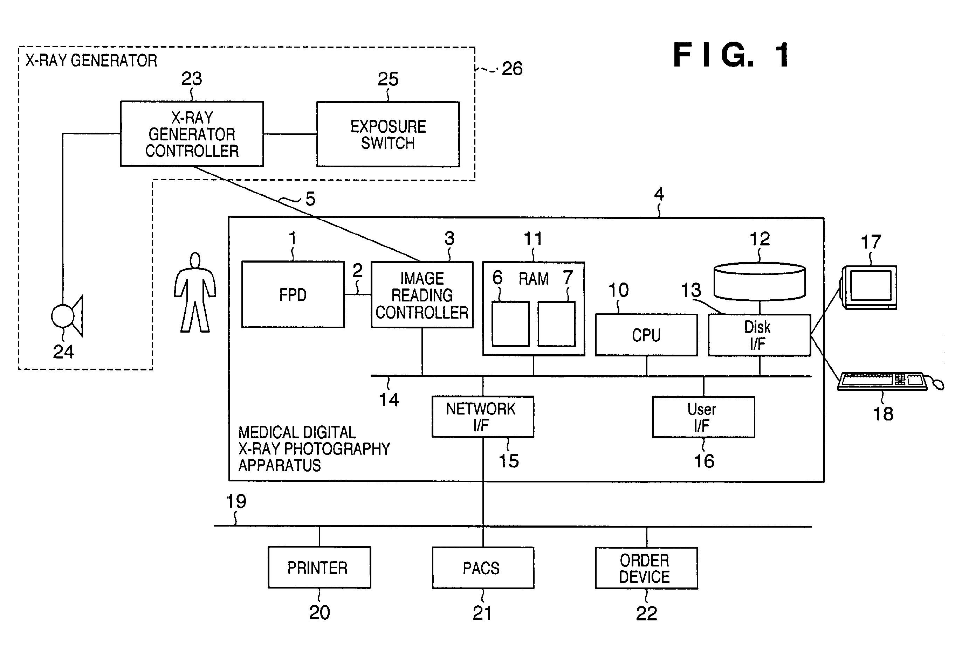

Fig. 1 is a block diagram illustrating the configuration of an X-ray image photography system according to an embodiment of the present invention. As shown in Fig. 1, the X-ray image photography system includes a medical digital X-ray photography apparatus 4, an X-ray generator 26, and various external devices connectable via a network 19 such as a LAN. The X-ray generator 26 comprises an X-ray tube 24, an X-ray generator controller 23 and an exposure switch 25.

The medical digital X-ray photography apparatus 4 comprises an FPD (Flat Panel Detector) 1, which is a sensor unit having a phosphor plate and a flat-panel optoelectronic transducer; an image reading controller 3 for synchronizing exposure with the X-ray generator 26; a CPU 10; a RAM 11 for storing a control program 6; a hard disk 12 for storing a captured image via a disk interface (DISK I/F) 13; a user interface (USER I/F) 16 for inputting and outputting information to and from interfaces operated by the operator; and a network interface 15 connectable to a network 19 (e.g., a communication LAN) connected to various external devices. Each of the components of the above-described medical digital X-ray photography apparatus 4 are interconnected via an internal bus 14 in the manner illustrated in Fig. 1.

The CPU 10 is a host CPU for executing the control program 6 of the invention in the medical digital X-ray photography apparatus 4. The RAM 11 stores the control program of the invention. The control program 6 is read in from the hard disk 12 and is run in the RAM 11 by the CPU 10. A RAM 7, on the other hand, is a RAM for temporarily storing the shot image held in RAM 11.

The FPD 1 and image reading controller 3 are connected to each other via a data line for power, image transfer and control signals. The hard disk 12 is a storage device for storing the program of the invention run by the CPU 10 and also functions to temporarily store correction information, which is necessary for photography, and captured images.

Examples of the external devices connected from the network interface 15 via the network 19 are a printer 20, a PACS (Picture Archiving/Communicating System) 21 and an order device 22, etc. More specifically, the network interface 15 is used to receive a shoot order from the external order device 22 and to transmit a shot image to the external devices such as the printer 20 and PACS 21 so as to be utilized in diagnosis.

Further, the user interface 16 sends and receives information to and from the operator of the medical digital X-ray photography apparatus 4 using various interfaces such as a display unit 17 and an input device 18 such as a keyboard and mouse. The display unit 17 and input device 18 may of course be replaced by a touch-sensitive panel.

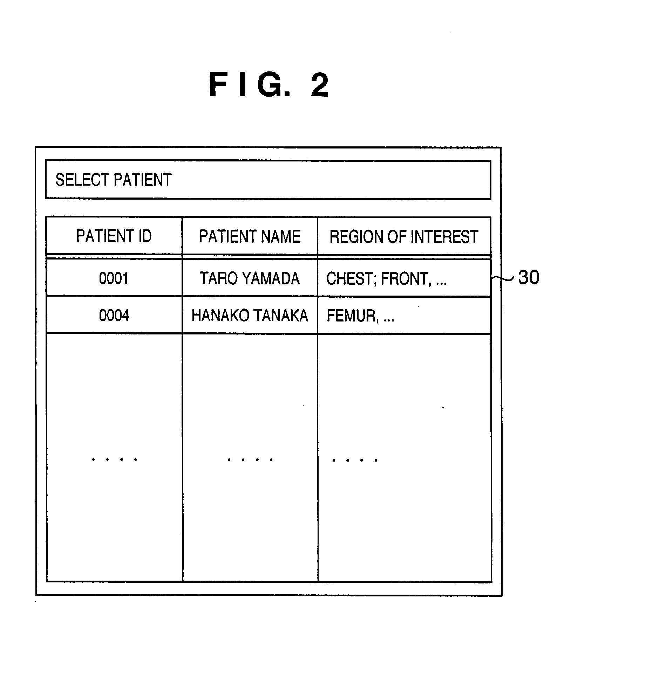

First, the CPU 10 of the medical digital X-ray photography apparatus 4 acquires ordering information from an RIS (Radiologic Information System), the information being received from the order device 22 on LAN 19. In this embodiment, a list of received ordering information is displayed on the display unit 17 from the user interface 16, as shown in Fig. 2. Fig. 2 is a diagram illustrating an example of display of a list of shoot orders displayed on the display unit 17 according to this embodiment. If a radiological technician, who is the operator of the medical digital X-ray photography apparatus 4, selects an examination indicated at numeral 30 in Fig. 2 using the input device 18 such as the mouse, then the screen displayed on the display unit 17 is changed by the control program 6 executed by the CPU 10, whereby detailed shoot-order information is displayed.

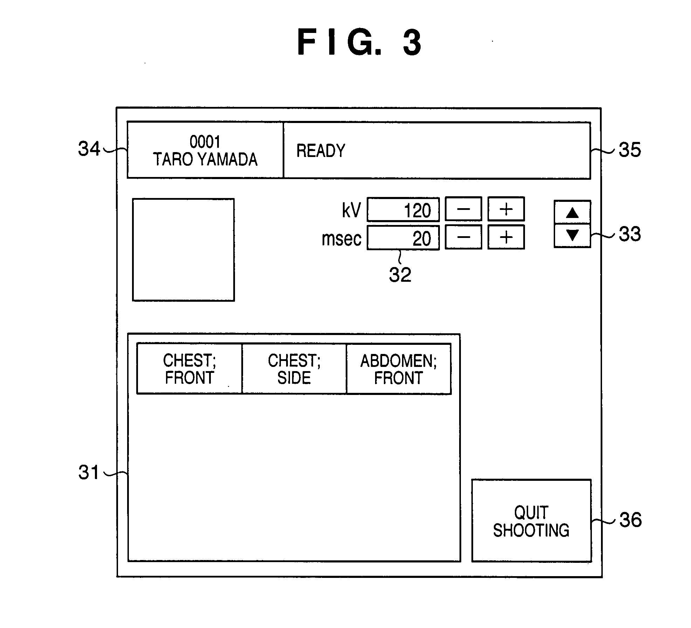

Fig. 3 is a diagram illustrating an example of display of a shoot preparation screen displayed on the display unit 17 according to this embodiment. For the patient indicated at numeral 30 selected on the screen shown in Fig. 2, the CPU 10 causes patient information 34, e.g., patient ID, name and date of birthday, etc., to be displayed on the screen. The shoot order, which is constituted by regions of interest to be shot and is received from the order device 22, is displayed in an area 31. The operator is capable of obtaining the above information based upon the screen display. Further, an area 32 shows the shooting conditions stored by a button representing the region of interest. A display section 35 displays the status of the medical digital X-ray photography apparatus 4.

Assume that shooting of the front of the chest first, the side of the chest second and the front of abdomen third has been specified as the ordering information, as illustrated in the shoot order 31. If a shooting operation starts at this time, the CPU 10 places a "CHEST; FRONT" button, which is the first region of interest of the ordering information indicated at 31, in the selected state. The operator places the patient to be exposed between the FPD 1 and the X-ray tube 24 and orients the patient to the posture for exposure. In the meantime, the CPU 10 applies voltage to the FPD 1 and prepares to perform X-ray photography using the FPD 1 in accordance with the control program 6. Next, when it is sensed that the FPD 1 is capable of performing X-ray photography, the control program 6 causes "READY", for example, to be displayed in the display section 35, as shown in Fig. 3.

After the operator has confirmed from the "READY" display that X-ray photography is possible, the operator presses the exposure switch 25 to input an X-ray-generating trigger to the system. An exposure signal generated by the exposure switch 25 is input to the image reading controller 3 via a synchronizing signal line 5. The image reading controller 3 causes an exposure-enable signal to be generated after it is confirmed from the status of a control signal 2 that the FPD 1 is in a shoot-enabled state. The exposure-enable signal is returned to the X-ray generator controller 23, which proceeds to cause the X-ray tube 24 to generate X rays.

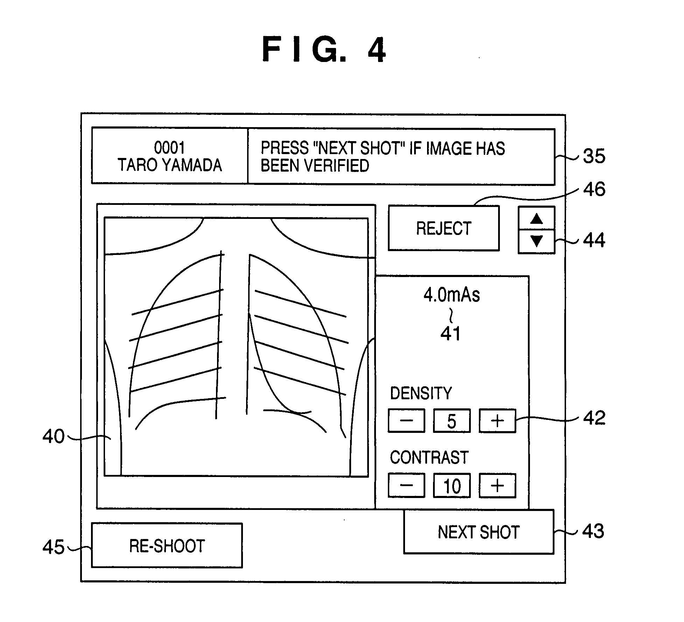

After irradiation with the X rays, the X rays that have passed through the patient are acquired as digital data by the FPD 1 via the phosphor plate and the digital data is transferred to the image reading controller 3. Next, the CPU 10 runs the control program 6 in such a manner that the display on the display unit 17 connected to the user interface 16 changes as shown in Fig. 4. Specifically, Fig. 4 is a diagram illustrating an example of a post-shoot display screen displayed on the display unit 17 according to this embodiment. As illustrated in Fig. 4, the image data that has been acquired from the image reading controller 3 is displayed in an area 40, and the CPU 10 receives a dose value 4 mAs from the X-ray generator controller 23 as shoot implementation information (Modality Performed Procedure Information), displays the value in an area 41 on the display unit 17 via the user interface 16 and stores the value on the hard disk 12.

Reference numeral 42 denotes an example of a parameter adjustment area for regulating density. For example, if the operator wants to alter the automatic density adjustment of the image displayed in area 40, then the operator can alter the density of the shot image by adjusting the parameter adjustment area 42. This adjustment may be performed by clicking on a "+" or "-" shown in Fig. 4 or by entering the parameter value directly.

After checking the shot image on the screen shown in Fig. 4, the operator, in accordance with display of a message "PRESS 'NEXT SHOT' IF IMAGE HAS BEEN VERIFIED" in display area 35, presses a "NEXT SHOT" button 43 on the screen to restore the screen of Fig. 3 in order to perform the next shooting operation. The CPU 10 allows the operator, through use of the input device 18 via the user interface 16, to select the next region of interest ordered and to repeat a similar shooting flow until all shoot orders have been completed. When all shoot orders have been completed, there is no next shot and therefore the operator can terminate shooting for examination purposes by pressing a button 36 in Fig. 3.

If shooting for examination purposes is terminated in the medical digital X-ray photography apparatus 4, then, in accordance with the control program 6, the CPU 10 sends the order device 22 the shooting conditions and shoot implementation information that has been stored in the hard disk 12. In accordance with a predetermined communication protocol, the control program 6 notifies the order device 22 of the fact that the shooting for examination has ended. At this time notification is given of the shooting conditions under which shooting took place and of shoot implementation information such as the radiation dose. Further, in accordance with a standard communication protocol for medical purposes referred to as DICOM, the control program 6 collectively outputs the shot image data, to which the shooting conditions and shoot implementation information have been appended as additional information, to the external devices so that the data may be used in making a diagnosis.

A method of shooting images in accordance with ordering information has been illustrated above. The purpose of examination is to acquire an image of a region of interest specified by a physician. However, there are occasions where, in actual shooting, success is not achieved by a single trial as in the manner described above. Often the reason for failure is blurring of the image caused by unintentional movement of the patient. This can lead to a so-called "rejected image", namely an image that is diagnostically useless.

In this case the operator re-shoots the rejected image. The shoot implementation information returned to the RIS does not require the rejected image that prevailed prior to re-shooting and the output image to the PACS does not also require the rejected image; it requires only a proper image in which the region of interest has been shot under the specified shooting conditions.

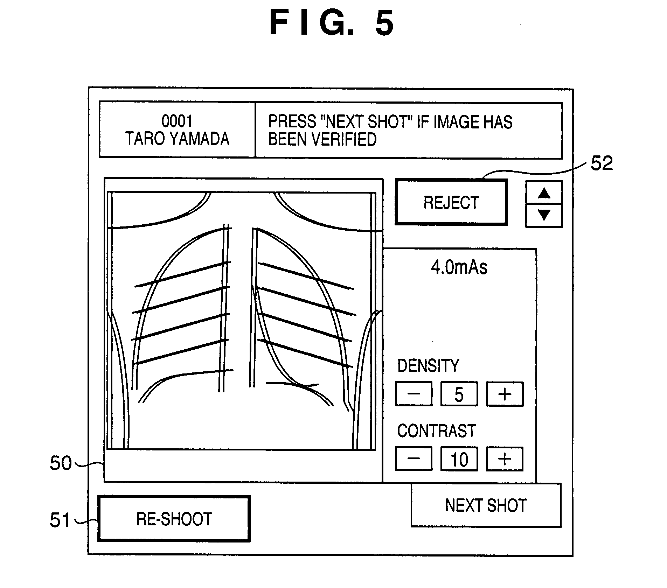

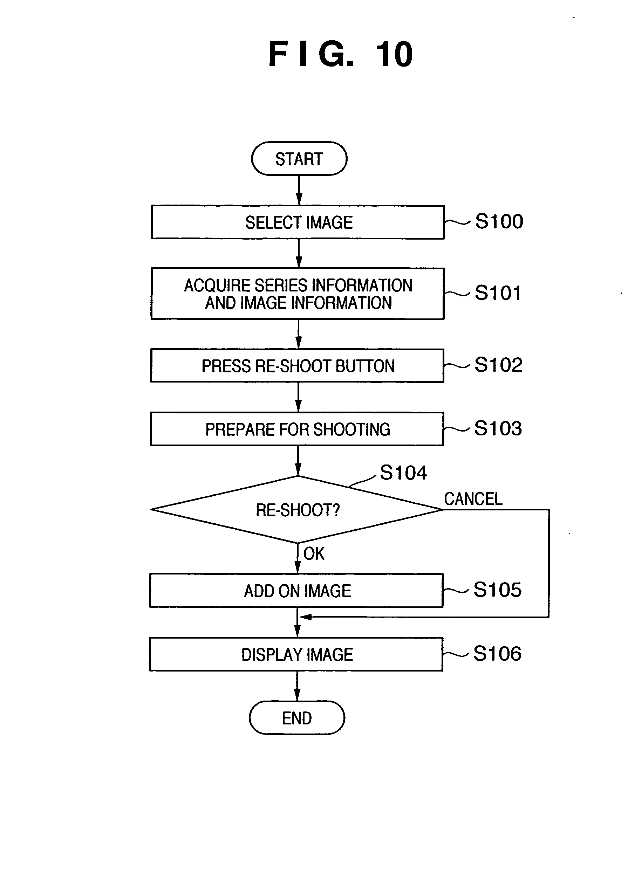

Accordingly, this embodiment will be described in regard to a case where re-shooting has become necessary owing to blurring of the shot image 50 in Fig. 5 caused by unintentional movement of the patient when the shot was taken. Fig. 5 is a diagram illustrating an example of a display screen showing the rejected image command-standby state by the operator because of confirming blurring of the shot image after shooting. Fig. 10 is a flowchart for describing the procedure of re-shoot processing in response to a re-shoot command.

First, a shot image that the operator has determined requires to be re-shot is selected (step S100) and information for managing the selected image is acquired (step S101). The operator then presses a re-shoot button 51 (step S102). In response, the FPD 1 is placed in the shoot-ready state again under the same conditions, the screen changes to the screen shown in Fig. 3 and the FPD 1, which is the sensor unit, is controlled and placed in a shoot-ready state (shoot-standby state) (step S103).

In re-shooting, the usual practice is to use the same shooting conditions, although the operator can alter the shooting conditions 32. It is then determined whether shooting has been performed (step S104). If the operator presses the exposure switch to cause generation of X rays, control proceeds to step S105. Here processing for adding on the image is executed. More specifically, all image information in a series for which re-shooting has been executed is searched successively and a validity flag is turned OFF. Further, newly added image information such as an image ID and shooting information is saved and a link from the rejected image is created. The details of processing for interchanging a rejected-image mark will be described later.

Next, control proceeds to step S106, where the image that is the result of re-shooting is displayed, as shown in Fig. 4. That is, if the operator presses the exposure switch 25 to perform re-shoot the image, the image obtained by this re-shooting is displayed as shown in Fig. 4 in a manner similar to that of the image that of the originally shot image. As a result, the image that was the object of re-shooting appears to the user automatically with a rejected-image mark attached thereto, and the original image that was rejected and the valid image shown in Fog. 4 can be checked and compared, as shown in Fig. 5, by pressing a shot-image display button 44. It should be noted that in a case where the operator presses a button 33 in Fig. 3 and "CANCEL" is selected instead of execution of re-shooting at step S104, control proceeds to step S106.

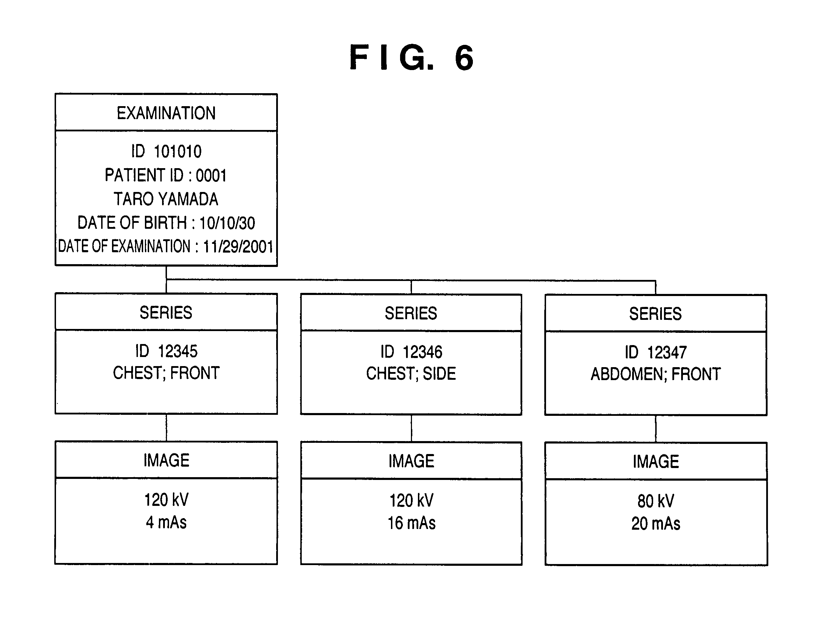

Here reference will be had to Figs. 6 to 8 to describe in detail a procedure through which the CPU 10, in response to execution of re-shooting, automatically performs control for replacing an already shot image with an image obtained by re-shooting.

Fig. 6 is a diagram useful in describing an example of image management before re-shooting by an ordinary digital photography apparatus according to the prior art. Specifically, a study attribute, series attribute and image attribute are illustrated as management information in Fig. 6. The control program 6 executed by the CPU 10 functions as an image management program and stores examination information as an examination ID 101010 when an examination starts. For example, in case of a shoot order for three shots, the program generates series information for every shot and causes one captured image to be stored for each shot.

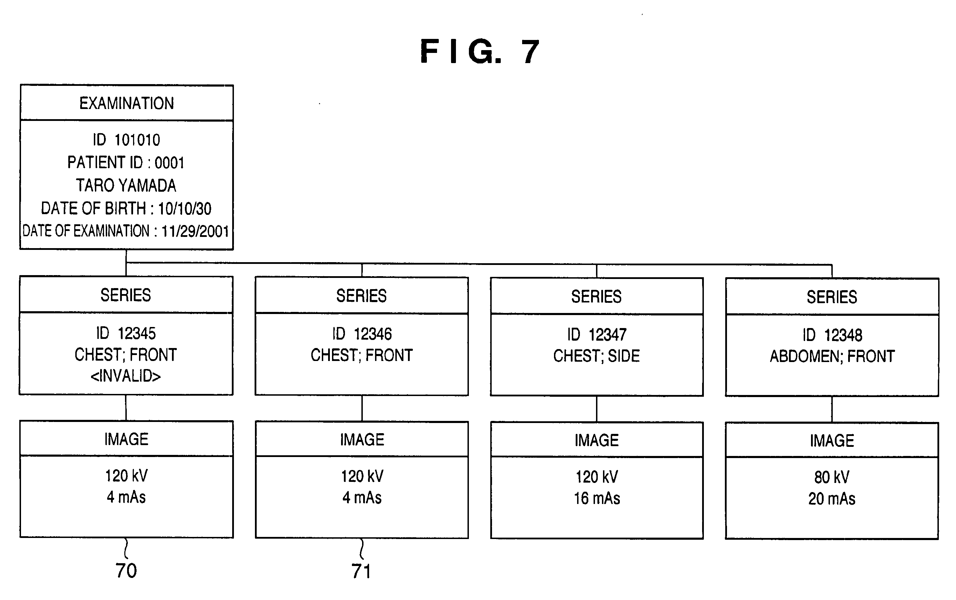

Fig. 7 is a diagram useful in describing an example of image management in a case where re-shooting has been performed with regard to "CHEST; FRONT" of a first shoot order. The front side of the chest is shot, a series ID 12345 constituting this image is generated and image management information 70 is stored. The first shot image is checked and the user determines that re-shooting is necessary. The operator therefore instructs the X-ray photography apparatus to perform re-shooting, thereby reproducing a series identical with that of the first stored image 70 and storing the results of re-shooting as well as image management information 71 that includes the image obtained by re-shooting. In this case, an invalidity flag is stored with respect to the series information having the ID 12345, whereby this series and image are treated as being rejected and control is carried out so as not to output the same to the external devices.

Further, when the display is presented, a rejected-image mark is added on in order to indicate that the image is an invalid image. Fig. 5 illustrates an example of display of an image stored as a rejected image automatically as a result of performing re-shooting in the manner described above. Reference numeral 52 denotes a rejected-image display area indicating that the image of the present shot is rejected. This indicates to the operator that this image is an invalid image that will not be output to external devices. A re-shoot button 51 is displayed in the non-selected state because re-shooting of a rejected image, that is, an invalid image is not allowed.

At certain hospitals, whether shots are successful or not is determined after successive shots have been taken as described above. If a failure has occurred owing to unintentional movement of the patient, the re-shoot button 45 is pressed to instruct the photography apparatus to perform re-shooting of an already shot image. In such case the images are arranged in the sequence of the order and therefore the images are output to the external devices in the sequence of the order when the examination ends. At other hospitals, however, there are instances where shooting is performed through a work flow wherein the button indicated at numeral 44 in Fig. 4 is pressed after the shooting of all orders in the sequence of orders 1, 2, 3 is completed, thereby returning the image to the leading image of the examination so that blurring of shot images can be checked one shot at a time and density adjusted one at shot at a time.

The button 44 in Fig. 44 is used to display shot images one after another. When the operator compares images by displaying them and removes a rejected-image mark from an image that had been marked as being rejected, this image is altered as a selected image and the validity flag thereof is turned ON. The image of the relevant series is retrieved and the validity flag of the already acquired image is turned OFF. That is, the rejected-image mark is appended to the image. In other words, control is exercised by the program in such a manner that only one certain selected image becomes the resultant image of this shoot order; other images are stored as rejected images.

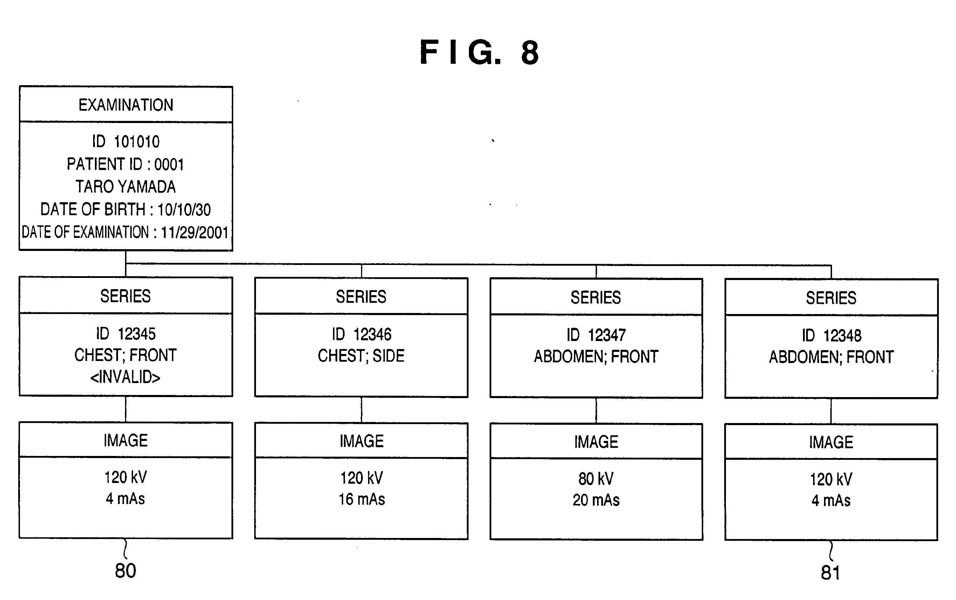

In this case, if the operator judges that the first shot needs to be re-shot, then the order for the first shot is re-shot as a fourth shot. Fig. 8 is a diagram useful in describing an example of image management in case of a fourth re-shoot with regard to "CHEST; FRONT" of a first re-shoot order. Specifically, a duplicate of series ID 12345 that includes a shot image 80 is created anew as series ID 12348, a re-shot image 81 is generated along with the re-shooting and this image is stored.

However, if implementation information is sent back to the order device 22 as is after re-shooting is performed, the sequence of the order and the operational result will change and this will cause difficulties at the order device 22. Furthermore, when an image captured by a first shot is compared with an image of a fourth shot that has been acquired by re-shooting, the operator's train of thought may be interrupted and it will be difficult to compare the two images if they are laid out far apart.

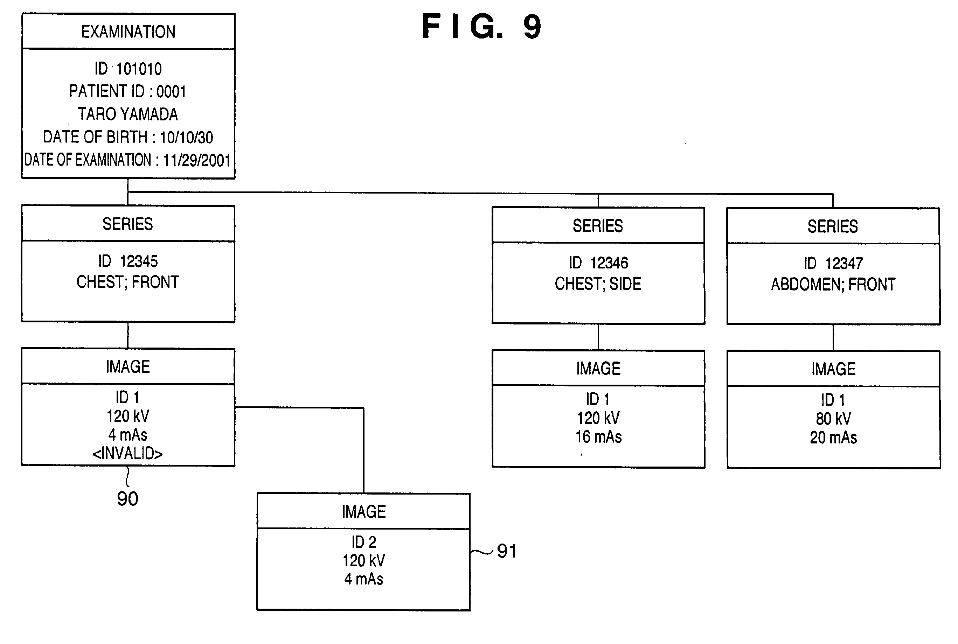

One possible method of solving this problem is to store separate ID information indicating the shoot order, rearrange the images shot by shot in accordance with this ID information and reflect this in the display. However, program processing for implementing this method is complicated. Accordingly, in this embodiment, as shown in Fig. 9, series information the number of items of which corresponds to the sequence of the order is created and, in case of re-exposure, image information is added to the applicable series as image ID 2 and the validity flag is turned ON for this image information. Fig. 9 is a diagram useful in describing an example of image management after re-shooting is performed according to this embodiment. Specifically, by turning OFF the validity flag of image information 90 and validating image information 91 linked to the image information 90, the implementation information can be returned to the order device 22 with the order sequence left as is. Adopting this expedient is advantageous in that rearrangement is unnecessary.

Furthermore, the image validity flag of the image information 90 is changed from OFF to ON. That is, if the rejected-image button 52 in Fig. 5 is pressed to restore validity, the valid image can be interchanged for this image merely by retrieving the link to the image information of the applicable series. Further, the validity flag of the image information 91 is changed to OFF to make the image a rejected image. By using this data structure according this embodiment, it is unnecessary to retrieve other series and program processing is facilitated.

More specifically, this embodiment is characterized in that shot images and re-shot images are stored on the hard disk 12 on a per-shoot-command basis (i.e., for every exposure order) using a hierarchical structure in which information relating to a shoot command (e.g., each series) is made a higher-order layer while the shot images and re-shot images are made a layer subordinate to the information relating to the shoot command, the shot and re-shot images being in the same layer as each other.

By adopting the arrangement described above, it is possible to realize a function in which if a shot fails, re-shooting is repeated and the optimum image selected from within the same order without re-inputting the image shooting conditions corresponding to the order. Further, by not sending an image having an invalidity flag from images in an examination to the external devices, only valid images the number of which is equivalent to the number of shots ordered are managed and these can be output to the external devices. Furthermore, since the images are laid out in correlated fashion (side by side), as shown in Fig. 9, in the sequence of the series constituting the orders, it is easy for the operator to compare valid images when re-shooting is executed.

Thus, as described above, management of shot images in an image sensing apparatus (the medical digital X-ray photography apparatus 4) according to this embodiment is characterized by acquiring a shot image of a subject that has been imaged, instructing the image sensing apparatus to re-shoot the image of the subject, acquiring the re-shot image of the subject that has been imaged by the image sensing apparatus, and storing the acquired shot image and the re-shot image on the hard disk 12 in correlated form. Management further includes adopting the shot image and the re-shot image that have been stored on the hard disk 12 as an invalid image and a valid image, respectively, and outputting only the valid image from the hard disk 12 to an external device or the like via, for example, a network.

Further, the image sensing apparatus is connected to the display unit 17, which displays shot images or re-shot images in the order of shoot commands. In a case where a plurality of images that include shot and re-shot images in a prescribed hierarchical layer situated subordinate to the same shoot command have been stored on the hard disk 12, the display unit 17 displays the plurality of images in order before it displays images relating to the next shoot command.

Further, according to this embodiment, an image is not written over another at the time of re-shoot and a rejected image is not erased, even when it is re-shot, but is stored within the storage device 12 shown in Fig. 1. Accordingly, when a rejected image other than an image used in diagnosis becomes necessary, it can be called and output again. A method of notifying the physician of this at the time of diagnosis is to store information indicating that re-shooting took place together with image information that is output to external devices, for example, header information in which the shooting conditions are stored.

More specifically, this embodiment is characterized in that if re-shooting of a subject has been specified, an invalidity mark is added to the originally shot image, and in that the shot image to which the invalidity mark has been added is stored on the hard disk 12 in correlation with the re-shot image. Further, this embodiment is characterized in that the shot image to which the invalidity mark has been added is adopted as an invalid image, the newly acquired re-shot image is adopted as a valid image and neither the invalid image nor the valid image are erased.

By way of example, if a transmission is made to the PACS 21, information indicating that re-shooting took place may be stored in a header beforehand and the information may be displayed when a display is presented on a display unit on the side of the PACS 21, and it is permissible to adopt an arrangement in which text or an icon is overlaid and displayed on the image. In case of output to the printer 20, text or an icon can be embedded in an image to an extent that will not hinder diagnosis and the resultant image can be output to the printer. In this embodiment, a physician who has viewed these displays can recognize that other images exist. This can cue the physician to contact the radiological technician and inquire about images that are rejected, and the technician can readily read rejected images out of the hard disk 12 again and output the rejected images again after selecting only them.

In other words, this embodiment is characterized by retaining a re-shoot flag indicative of existence of an associate invalid image as information (e.g., a header) appended to shot and re-shot images.

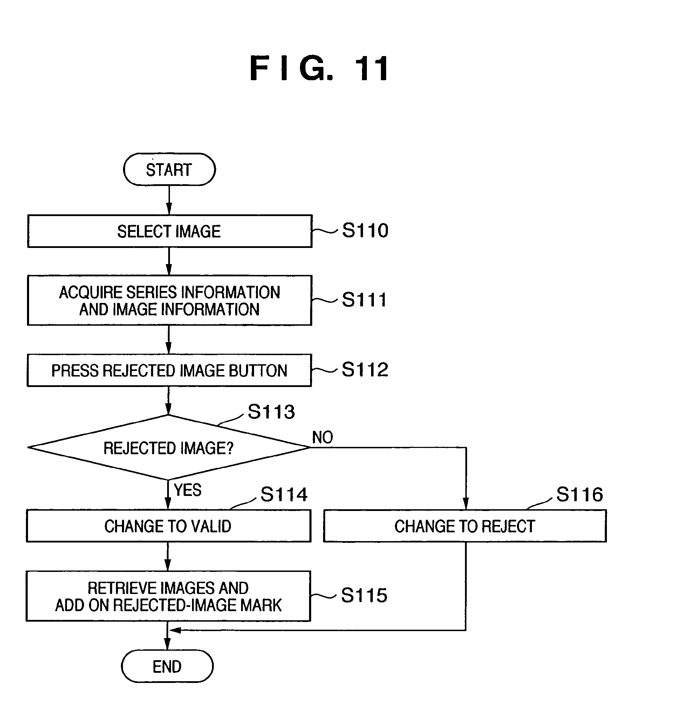

Fig. 11 is a flowchart for describing the procedure of image management in response to operation of a rejected-image button 46. First, as described above, an image that has already been shot is selected (step S110) and information for managing the selected shot image is acquired (step S111). Next, it is determined that the rejected-image button 46 has been pressed (step S112) and then it is determined whether the present image information is in a state indicative of a rejected image (step S113). If the result is that the present image information indicates a rejected image ("YES" at step S113), then the image information is changed to valid (flag ON) (step S114). By retrieving the other images in the series and turning validity flags OFF, a change is made to rejected (step S115). On the other hand, if a rejected image is not indicated at step S113, that is, of the image is valid ("NO" at step S113), then the validity flag of this image is turned OFF to change the image to a rejected image (step S116).

The embodiment above has been described in regard to a case where a control program is stored on a hard disk, transferred to a RAM and executed. However, the invention is not limited to such an arrangement and may be implemented using any storage medium. Further, the invention may be implemented using circuits that operate in a manner similar to the control program.

By using an X-ray image photography system according to this embodiment, as described above, an image which re-shoot has been commanded is treated as a rejected image automatically without affecting hospital work flow and wasteful film output can be prevented without the awareness of the operator. Further, it is easy to compare a valid image and a rejected image in a shoot order and an image suitable for diagnosis can be chosen from among a group of shot images. Furthermore, images can be sent back to the order device or the sequence of image output to external devices controlled without the labor involved in rearranging images in the sequence of the shoot orders.

Furthermore, by storing in output image information the fact that re-shooting has been performed, the person making the diagnosis can be made to be aware of the fact that other images exist. As a result, in a case where an image that has been delivered to a physician is not acceptable and does not easily lend itself to diagnosis, the image makes the physician aware of the fact that a shooting failure took place so that the physician can make an overall judgement by using the rejected image as well. Further, according to this embodiment, wasteful film output can be prevented even without the operator being aware.

In other words, in accordance with this embodiment, as described above, it may be arranged that information indicating that a valid image is a re-shot image is added onto the valid image. Further, it may be so arranged that when a valid image is displayed on the display unit 17, auxiliary information indicating the absence or presence of an invalid image corresponding to the valid image is displayed in the valid image or together with the display of the valid image.

Note that the present invention can be applied to an apparatus characterized by comprising a single device or to system constituted by a plurality of devices.

Furthermore, the invention can be implemented by supplying a software program, which implements the functions of the foregoing embodiments, directly or indirectly to a system or apparatus, reading the supplied program code with a computer of the system or apparatus, and then executing the program code. In this case, so long as the system or apparatus has the functions of the program, the mode of implementation need not rely upon a program.

Accordingly, since the functions of the present invention are implemented by computer, the program code installed in the computer also implements the present invention. In other words, the claims of the present invention also cover a computer program for the purpose of implementing the functions of the present invention.

In this case, so long as the system or apparatus has the functions of the program, the program may be executed in any form, such as an object code, a program executed by an interpreter, or scrip data supplied to an operating system.

Example of storage media that can be used for supplying the program are a floppy disk, a hard disk, an optical disk, a magneto-optical disk, a CD-ROM, a CD-R, a CD-RW, a magnetic tape, a non-volatile type memory card, a ROM, and a DVD (DVD-ROM and a DVD-R).

As for the method of supplying the program, a client computer can be connected to a website on the Internet using a browser of the client computer, and the computer program of the present invention or an automatically-installable compressed file of the program can be downloaded to a recording medium such as a hard disk. Further, the program of the present invention can be supplied by dividing the program code constituting the program into a plurality of files and downloading the files from different websites. In other words, a WWW (World Wide Web) server that downloads, to multiple users, the program files that implement the functions of the present invention by computer is also covered by the claims of the present invention.

It is also possible to encrypt and store the program of the present invention on a storage medium such as a CD-ROM, distribute the storage medium to users, allow users who meet certain requirements to download decryption key information from a website via the Internet, and allow these users to decrypt the encrypted program by using the key information, whereby the program is installed in the user computer.

Besides the cases where the aforementioned functions according to the embodiments are implemented by executing the read program by computer, an operating system or the like running on the computer may perform all or a part of the actual processing so that the functions of the foregoing embodiments can be implemented by this processing.

Furthermore, after the program read from the storage medium is written to a function expansion board inserted into the computer or to a memory provided in a function expansion unit connected to the computer, a CPU or the like mounted on the function expansion board or function expansion unit performs all or a part of the actual processing so that the functions of the foregoing embodiments can be implemented by this processing.

In accordance with the present invention, as described above, shot images can be managed in ideal fashion without outputting unnecessary film even in a case where re-shooting is performed a number of times with regard to a shoot order.

Further, an image that has been captured by re-shooting is stored in correlation with the original image of the re-shoot and the sequence of images can be displayed ideally irrespective of the shooting sequence.

Furthermore, by incorporating in header information the fact that re-shooting has been performed, it is possible to make the operator aware of the presence of a rejected image that corresponds to a valid image.

The present invention is not limited to the above embodiments and various changes and modifications can be made within the spirit and scope of the present invention. Therefore, to apprise the public of the scope of the present invention, the following claims are made.

Disclosed are an image management method, apparatus and program through which shot images can be managed in ideal fashion without outputting unnecessary film even in a case where re-shooting is performed a number of times in regard to a shoot order. Management of shot images in a medical digital X-ray photography apparatus (4) includes acquiring a shot image of an imaged subject, instructing the image sensing device to re-shoot the image of the subject, acquiring the re-shot image of the subject that has been imaged by the image sensing apparatus, and storing the acquired shot image and the re-shot image on a hard disk (12) in correlated form. Management further includes adopting the shot image and the re-shot image that have been stored on the hard disk (12) as an invalid image and a valid image, respectively, and outputting the valid image from the hard disk (12) to an external device or the like.

## Claims

1. An image management method **characterized by** comprising:
a first acquisition step of acquiring a shot image of a subject that has been imaged by an image sensing device;
an command step of commanding the image sensing device to re-shoot the image of the subject;
a second acquisition step of acquiring the re-shot image of the subject imaged by the image sensing device;
a storage step of storing the shot image, which has been acquired at said first acquisition step, and the re-shot image, which has been acquired by said second acquisition step, in correlated form in a storage device;
a status setting step of adopting the shot image and the re-shot image, which have been stored in the storage device, as an invalid image and a valid image, respectively; and
an output step of outputting the valid image only from the storage device.

2. The method according to claim 1, **characterized in that** said storage step stores the shot and re-shot images in the storage device on a per-shoot-command basis at said command step using a hierarchical structure in which information relating to the shoot command is made a higher-order layer while the shot image and re-shot image are made a layer subordinate to information relating to the shoot command, the shot and re-shot images being in the same layer as each other.

3. The method according to claim 1 or 2, **characterized by** further comprising a display step of displaying the shot image or re-shot image in the order of shoot commands at said command step.

4. The method according to claim 3, **characterized in that** in a case where a plurality of images that include shot and re-shot images in a prescribed hierarchical layer situated subordinate to the same shoot command have been stored in the storage device, said display step displays the plurality of images in order before images relating to the next shoot command or the shoot sequence are displayed.

5. The method according to any one of claims 1 to 4, **characterized in that** said status setting step causes an invalidity flag indicative of an invalid image or a validity flag indicative of a valid image to be retained as information attached to the shot and re-shot images.

6. The method according to any one of claims 1 to 5, **characterized by** further comprising a mark add-on step of adding an invalidity mark onto the shot image if re-shooting of the subject has been command by said command step;
**characterized in that** said storage step stores the shot image having the added-on invalidity mark in correlation with the re-shot image.

7. The method according to claim 6, **characterized in that** said status setting step adopts the shot image having the added-on invalidity mark as an invalid image, adopts the re-shot image, which has been acquired by said second acquisition step, as a valid image, and erases neither the invalid image nor the valid image.

8. The method according to any one of claims 1 to 7, **characterized by** further comprising an information add-on step of adding on information, which indicates that the valid image is a re-shot image, to the valid image.

9. The method according to claim 1, **characterized by** further comprising an auxiliary-information display step which, when the valid image is displayed by said display step, is a step of displaying auxiliary information, which indicates the absence or presence of an invalid image corresponding to the valid image, in the valid image or together with the display of the valid image.

10. An image management apparatus **characterized by** comprising:
shooting means for shooting a subject and acquiring a shot image of the subject;
command means for commanding the image sensing device to re-shoot the image of the subject;
re-shooting means for re-shooting the subject and acquiring a re-shot image of the subject based upon a command from said command means;
storage means for storing the shot image and the re-shot image in correlated form;
status setting means for adopting the shot image and the re-shot image, which have been stored in said storage means, as an invalid image and a valid image, respectively; and
output means for outputting the valid image from said storage device.

11. A program for causing a computer to execute the following procedures:
a first acquisition procedure for acquiring a shot image of a subject that has been imaged by an image sensing device;
an command procedure for commanding the image sensing device to re-shoot the image of the subject;
a second acquisition procedure of acquiring the re-shot image of the subject imaged by the image sensing device;
a storage procedure for storing the shot image, which has been acquired at said first acquisition step, and the re-shot image, which has been acquired by said second acquisition procedure, in correlated form in a storage device;
a status setting procedure for adopting the shot image and the re-shot image, which have been stored in the storage device, as an invalid image and a valid image, respectively; and
an output procedure for outputting the valid image from the storage device.

12. A computer-readable recording medium on which the program set forth in claim 11 has been stored.
